# EUROPÄISCHE PATENTSCHRIFT

(11) **EP 3 260 162 B1**
(45) Veröffentlichungstag und Bekanntmachung des Hinweises auf die Patenterteilung: **18.09.2019**
(21) Anmeldenummer: 16175951.9
(22) Anmeldetag: 23.06.2016
(51) Int. Cl.: A61M 39/22

(54) **AUTOMATISCHE VENTILBRECHVORRICHTUNG MIT DOKUMENTATION UND STEUERUNG DES BRECHVORGANGES**
AUTOMATIC VALVE BREAKING DEVICE WITH DOCUMENTATION AND CONTROL OF THE BREAKING PROCESS
DISPOSITIF DE RUPTURE DE SOUPAPE AUTOMATIQUE AVEC DOCUMENTATION ET CONTRÔLE DU PROCESSUS DE RUPTURE

(43) Veröffentlichungstag der Anmeldung: 27.12.2017
(73) Patentinhaber: LMB Technologie GmbH, 85445 Schwaig (DE)
(72) Erfinder: Jentsch, Klaus, 85445 Schwaig (DE)
(74) Vertreter: Winter, Brandl, Fürniss, Hübner, Röss, Kaiser, Polte - Partnerschaft mbB

(56) Entgegenhaltungen:
- WO-A1-2016/078872
- DE-U1-202011 110 763
- US-A1- 2010 132 512
- US-A1- 2014 263 529

## Beschreibung

Die vorliegende Erfindung betrifft eine automatische Ventilbrechvorrichtung zum Brechen eines Brechventils einer medizinischen Schlauchleitung, bei der der Ventilbrechvorgang mittels einer Kamera gesteuert, überwacht und dokumentiert wird.

Zudem betrifft die vorliegende Erfindung ein Verfahren zum Brechen eines Brechventils einer medizinischen Schlauchleitung, bei dem der Erfolg des Brechvorgangs durch eine automatische Überwachung des Brechvorgangs mittels einer Kamera und eine entsprechende Ansteuerung der Ventilbrechvorrichtung sichergestellt wird, sodass eine Überwachung bzw. ein Eingreifen beispielsweise durch medizinisches Fachpersonal nicht mehr benötigt wird.

### Stand der Technik

Medizinische Schlauchleitungen, wie beispielsweise die an Blutbeutel, Infusionsbeutel oder sonstige Behältnisse medizinischer Lösungen konnektierten Schläuche oder Leitungen, sind zur Abdichtung und Gewährleistung der Sterilität der enthaltenen medizinischen Lösungen herkömmlicherweise mit Brechventilen bzw. Brechsiegeln ausgestattet.

Zum Öffnen der jeweiligen Fließstrecke einer medizinischen Lösung in einer derartigen Schlauchleitung muss daher zunächst das Brechventil gebrochen werden. Herkömmlicherweise weisen derartige Brechventile hierfür eine Sollbruchstelle auf, an welcher das Brechventil durch Knicken gebrochen wird.

Das Brechen des Brechventils kann händisch erfolgen, hierbei kann beispielsweise eine Krankenschwester vor dem Start einer Infusion das Brechventil durch Knicken brechen und somit den Fließweg für die jeweilige Infusionslösung öffnen. Ein derartiges händisches Brechen des Brechventils ist jedoch insbesondere bei einer wiederholten Durchführung des Brechvorgangs für die Hände des klinischen Personals sehr belastend.

Daher besteht ein Bedarf nach Hilfsmitteln, um den Brechvorgang des Brechventils schonender zu gestalten und eventuell sogar zu automatisieren.

US 2014 / 0 263 529 A1 lehrt daher die Verwendung einer Ventilbrechvorrichtung, die eine drehbare Aufnahme und Stifte aufweist, wobei ein zu brechendes Ventil zwischen der drehbaren Aufnahme und den Stiften angeordnet ist. Das Ventil wird anschließend durch ein Hin- und Herbewegen der drehbaren Aufnahme um einen bestimmten Winkel gebrochen.

US 2010 / 0 132 512 A1 offenbart eine tragbare Vorrichtung, die verwendet wird, um ein zu brechendes Ventil eines Blutbeutels, der in einer Vorrichtung aufgenommen ist, zu brechen. Aus diesem Grund benötigt die tragbare Vorrichtung zwei Achsen, um den in der Vorrichtung angeordneten Blutbeutel zu erreichen. Zudem muss eine Kontrolle der vollstängien Brechung weiterhin manuell erfolgen.

WO 2016 / 078 872 A1 offenbart eine Vorrichtung, mit der ein Ventil auf zwei unterschiedliche Weisen gebrochen werden können. Bei einer der beiden Brechanordnungen werden zwei Aufnahmen zueinander gedreht, so dass das dazwischen angeordnete zu brechende Ventil gebrochen wird. Dabei ist es jedoch erforderlich, dass dazu auch die erste Brechanordnung angetrieben wird.

DE 20 2011 110 763 U1 offenbart eine weitere Vorrichtung zum Brechen eines Ventils, die eine feststehende und eine bewegliche Aufnahme aufweist, wobei ein Bruch eines Ventils durch Versetzen des beweglichen Elements bewirkt wird.

Allerdings steht hierbei die Gewährleistung der Patientensicherheit an erster Stelle, denn mit einem Wegfall der Erfordernis, dass das Brechventil von klinischem Personal gebrochen wird und daher zwingend der direkten Kontrolle durch das klinische Personal unterliegt, darf keine Verringerung der Gewährleistungssicherheit einhergehen, mit der das Brechventil korrekt, d.h. vollständig, gebrochen wird.

Der vorliegenden Erfindung liegt daher die Aufgabe zugrunde, den Vorgang des Brechens eines derartigen Brechventils zu vereinfachen, ohne dass die Patientensicherheit gefährdet wird.

Diese Aufgabe wird durch die Ventilbrechvorrichtung mit den Merkmalen gemäß Anspruch 1 und durch das Verfahren zum Brechen eines Brechventils mit den Schritten gemäß Anspruch 12 gelöst.

### Kurzbeschreibung der Erfindung

Eine erfindungsgemäße Ventilbrechvorrichtung zum Brechen eines Brechventils / Brechsiegels einer medizinischen Schlauchleitung hat ein erstes Drehelement, welches relativ zu einer zentralen Longitudinalachse der Ventilbrechvorrichtung bewegbar ist, und ein zweites Drehelement, welches relativ zu der zentralen Longitudinalachse der Ventilbrechvorrichtung bewegbar ist und welches mit dem ersten Drehelement derart in einer Wirkverbindung steht, dass eine Bewegung des ersten Drehelements in einer ersten Richtung eine Bewegung des zweiten Drehelements in einer zu der ersten Richtung gegenläufigen zweiten Richtung bewirkt, und ein Aufzeichnungsmittel zur Überwachung und Aufzeichnung des Vorgangs des Brechens des Brechventils.

Der Kerngedanke der Erfindung besteht hierbei darin, das Brechventil durch die gegenläufige Bewegung des ersten und zweiten Drehelements zu brechen. Durch die gegenläufige Drehbewegung der beiden Drehelemente wird das Brechventil abgeknickt. Vorteilhafterweise können hierbei das erste und das zweite Drehelement jeweils eine Aufnahme aufweisen, in welcher die Schlauchleitung mit dem zu brechenden Brechventil kraftschlüssig, reibschlüssig und / oder formschlüssig gehalten werden kann.

Die erfindungsgemäße Ventilbrechvorrichtung kann auch als Siegelbrechvorrichtung verstanden werden, da das zu brechende "Ventil" nicht zwingend einen einstellbaren Strömungsquerschnitt aufweisen muss, sondern nach Art eines Siegels lediglich digital zwischen einem Offen- und einem Geschlossen-Zustand umgeschaltet werden kann.

Beispielsweise kann das zu brechende Brechventil bzw. Brechsiegel ein die Sterilität einer medizinischen Flüssigkeit im Rahmen einer Einmal-Verpackung (z.B. eines Blutbeutels) gewährleistendes Siegel sein, welches lediglich einmalig zur Verwendung der medizinischen Flüssigkeit gebrochen wird, woraufhin die leere Einmal-Verpackung entsorgt wird.

In diese Aufnahmen wird die Schlauchleitung mit dem zu brechenden Brechventil eingesetzt, vorteilhafterweise erstreckt sich die Schlauchleitung hierbei entlang ihrer eigenen Longitudinalachse und auch entlang der Longitudinalachse der Ventilbrechvorrichtung.

Um das Brechventil zu brechen, wird nun das erste und / oder das zweite Drehelement der Ventilbrechvorrichtung derart bewegt, dass das zu brechende Brechventil in der Schlauchleitung zumindest abschnittsweise aus seiner Position entlang der Longitudinalachse des Brechventils bewegt wird. Dies erfolgt beispielsweise durch eine Auslenkung bzw. Verschwenkung des ersten und / oder zweiten Drehelements der Ventilbrechvorrichtung weg von der Longitudinalachse der Ventilbrechvorrichtung.

Vorteilhafterweise werden das erste und das zweite Drehelement in einander gegenläufigen Richtungen weg von der Longitudinalachse der Ventilbrechvorrichtung verschwenkt bzw. ausgelenkt, d.h. das erste Drehelement wird beispielsweise im Uhrzeigersinn ausgelenkt und das zweite Drehelement wird beispielsweise im Gegenuhrzeigersinnn ausgelenkt, oder umgekehrt. Der Drehpunkt eines jeden der beiden Drehelemente kann sich hierbei beliebig im Zentrum des jeweiligen Drehelements befinden, oder auch von diesem Zentrum verschoben sein.

Durch die gegenläufige Bewegung des ersten und zweiten Drehelements wird das zu brechende Brechventil gebrochen.

Gemäß einem gegebenenfalls separat zu beanspruchenden Aspekt der Erfindung weist die Ventilbrechvorrichtung zudem ein Begrenzungsmittel auf, welches die Bewegung des ersten und / oder des zweiten Drehelements begrenzt. Ein derartiges Begrenzungsmittel kann beispielsweise die Form eines Anschlags / Kontaktpunkts haben, durch welchen eine über einen im Vorhinein eingestellten Maximalwert hinausgehende Bewegung des ersten und / oder des zweiten Drehelements in einer definierten Richtung begrenzt bzw. beschränkt wird. Zum Beispiel könnte mittels dieses Begrenzungsmittels der Bewegungsbereich des ersten Drehelements auf eine Auslenkung von 0° bis 70° relativ zu der Longitudinalachse der Ventilbrechvorrichtung eingeschränkt werden. Vorzugsweise liegt der Bewegungsbereich mindestens des ersten Drehelements 4 zwischen ca. 0° und ca. 90°, vorzugsweise zwischen ca. 0° und ca. 70°, insbesondere zwischen ca. 0° und ca. 60°, wobei die Gradzahl den Winkel einer Auslenkung der Longitudinalachse des ersten Drehelements relativ zu der Longitudinalachse der gesamten Ventilbrechvorrichtung und / oder relativ zu der Longitudinalachse einer im Ruhezustand in eine erfindungsgemäße Ventilbrechvorrichtung eingeführten Schlauchleitung angibt.

Gemäß einem gegebenenfalls separat zu beanspruchenden Aspekt der Erfindung ist das erste Drehelement aktiv bewegbar und das zweite Drehelement ist durch die Bewegung des ersten Drehelements passiv bewegbar. In anderen Worten kann das erste Drehelement aktiv, bevorzugt beispielsweise durch einen Motor, aber grundsätzlich auch durch eine händische Betätigung durch einen Benutzer, bewegt werden, und auf das zweite Drehelement muss nicht separat eingewirkt werden, es wird passiv allein durch die Wirkverbindung zwischen dem ersten Drehelement und dem zweiten Drehelement mitbewegt.

Konkret kann gemäß einem gegebenenfalls separat zu beanspruchenden Aspekt der Erfindung vorteilhafterweise die Wirkverbindung zwischen dem ersten und dem zweiten Drehelement ein Zahnradeingriff / zwei ineinander eingreifende Zahnradabschnitte sein, sodass durch eine aktive Bewegung des ersten Drehelements das zweite Drehelement passiv bzw. ohne eine zweite aktive direkte Einwirkung mitbewegt wird.

Um Sicherzustellen, dass das Brechventil korrekt, d.h. vollständig gebrochen ist, weist die Ventilbrechvorrichtung zudem ein Aufzeichnungsmittel zur Qualitätskontrolle auf. Das Aufzeichnungsmittel ermöglicht die Überwachung des Brechens des Brechventils und bietet zudem die Möglichkeit der Dokumentation, sodass im Nachhinein belegt werden kann, dass das Brechventil vollständig gebrochen worden ist.

Das Aufzeichnungsmittel kann beispielsweise eine Kamera zur optischen Erfassung des Brechvorgangs sein, grundsätzlich wären aber auch andere Aufzeichnungsmodalitäten, wie z.B. eine akustische Aufzeichnung des Brechvorgangs (Knackgeräusch bei einem erfolgreichen Brechen des Brechventils) möglich.

Das Aufzeichnungsmittel kann nur eine einzige Kamera oder nur ein einziges sonstiges Aufzeichnungsmittel sein, das Aufzeichnungsmittel kann aber auch eine Mehrzahl an Kameras oder eine Mehrzahl an sonstigen Aufzeichnungsmitteln sein.

Gemäß einem weiteren vorteilhaften, gegebenenfalls separat zu beanspruchenden Aspekt der Erfindung ist das Aufzeichnungsmittel zwischen und / oder neben dem ersten und dem zweiten Drehelement vorzugsweise auf der Höhe des Wirkeingriffs angeordnet. Der Aufzeichnungsbereich des Aufzeichnungsmittels erfasst somit optimal den Abschnitt des Brechventils, in welchem das Brechventil gebrochen wird, z.B. an der Sollbruchstelle.

Das Aufzeichnungsmittel zeichnet bevorzugt optische Daten bezüglich des Vorgangs des Brechens des Brechventils auf, welche daraufhin mit im Vorhinein bestimmten Referenzdaten abgeglichen werden, wodurch bestimmt wird, ob das Brechventil vollständig gebrochen ist.

Konkret kann dies dadurch erfolgen, dass das Aufzeichnungsmittel das Brechventil bildlich überwacht und die aufgezeichneten Bilddaten daraufhin an eine Vergleichseinheit weitergegeben werden, in welcher diese Bilddaten jeweils mit im Vorhinein gespeicherten Aufnahmen eines vollständig gebrochenen Brechventils gleichen Typs als Referenzdaten abgeglichen werden. Die Vergleichseinheit kann beispielsweise in einer Steuerungseinheit realisiert sein. Dieser Bildabgleich ermöglicht die Durchführung einer Bestimmung, ob das zu brechende Brechventil vollständig gebrochen ist.

Gemäß einem weiteren vorteilhaften, gegebenenfalls separat zu beanspruchenden Aspekt der Erfindung weist die Ventilbrechvorrichtung zudem eine Steuerungseinheit auf, welche dazu ausgelegt ist, eine Bewegung mindestens des ersten Drehelements abzubrechen, wenn durch den Abgleich der von dem Aufzeichnungsmittel ermittelten Daten mit den Referenzdaten bestimmt worden ist, dass das Brechventil vollständig gebrochen ist; oder eine Bewegung mindestens des ersten Drehelements weiterzuführen, wenn durch den Abgleich der von dem Aufzeichnungsmittel ermittelten Daten mit den Referenzdaten bestimmt worden ist, dass das Brechventil unvollständig gebrochen ist.

Die Steuerungseinheit führt somit bevorzugt eine Rückkoppelungssteuerung der Bewegung mindestens des ersten Drehelements durch auf der Grundlage der Bestimmung, ob das zu brechende Brechventil vollständig gebrochen ist.

Beispielsweise wird, während das Aufzeichnungsmittel kontinuierlich die Sollbruchstelle des Brechventils überwacht und aufzeichnet, das erste Drehelement über einen von der Steuerungseinheit gesteuerten Motor solange und soweit in eine erste Richtung verschwenkt / ausgelenkt, bis das Brechventil vollständig gebrochen ist. Die Aufzeichnung durch das Aufzeichnungsmittel kann auch nur intermittierend und / oder getaktet zu diskreten Zeitpunkten stattfinden.

Sobald das Brechventil vollständig gebrochen ist, zeichnet das Aufzeichnungsmittel ein Bild des vollständig gebrochenen Brechventils auf und der darauf folgende Bildabgleich des aufgezeichneten Bildes mit einem in den Referenzdaten enthaltenen Bild eines vollständig gebrochenen Brechventils gleichen Typs führt zu einer Bestimmung, dass das Brechventil nun vollständig gebrochen ist. Da bestimmt worden ist, dass das Brechventil vollständig gebrochen ist, ist keine weitere Auslenkung / Verschwenkung mindestens des ersten Drehelements notwendig und die Bewegung, d.h. die Auslenkung / Verschwenkung mindestens des ersten Drehelements wird abgebrochen bzw. gestoppt.

Da durch die Bewegung des ersten Drehelements vorzugsweise die gegenläufige Bewegung des zweiten Drehelements bewirkt wird, muss lediglich die Bewegung des ersten Drehelements aktiv gesteuert, d.h. gestoppt oder weitergeführt werden.

Daher kann es ausreichend sein, wenn nur das erste Drehelement von der Steuerungseinheit angesteuert wird.

Wenn jedoch der Bildabgleich des aufgezeichneten Bildes mit einem in den Referenzdaten enthaltenen Bild eines vollständig gebrochenen Brechventils gleichen Typs zu einer Bestimmung führt, dass das Brechventil nicht vollständig gebrochen ist, so wird die Bewegung, d.h. die Auslenkung / Verschwenkung mindestens des ersten Drehelements in der ersten Richtung solange und soweit weitergeführt bzw. beibehalten, bis einen weiterer Bildabgleich zu einer positiven Bestimmung führt, dass das Brechventil vollständig gebrochen ist.

Somit kann gewährleistet werden, dass das erste Drehelement immer gleich bei dem ersten Versuch des Brechens des Brechventils ausreichend ausgelenkt wird, sodass das Brechventil vollständig gebrochen wird. Bei einem herkömmlichen, insbesondere einem rein händischen Brechen eines Brechventils, musste das Brechventil häufig mehrfach geknickt werden, bis das Brechventil vollständig gebrochen war. Häufig muss das Brechventil nämlich stärker geknickt werden, als anfangs gedacht. Durch die mehrfachen Versuche des Brechens des Brechventils geht jedoch wertvolle Zeit verloren, während der beispielsweise zu behandelndes Blut in einem Blutbeutel vor der Verarbeitung schädigenden Umwelteinflüssen ausgesetzt ist, oder während der ein Patient auf eine wichtige Infusion warten muss. Zudem können unvollständig gebrochene Brechventile zu einer unerwünschten Haemolyse bei der Blutaufbereitung führen.

All diese Nachteile werden behoben, wenn das Brechventil mit einer erfindungsgemäßen Ventilbrechvorrichtung bereits beim ersten Versuch, schnell, effizient und sicher vollständig gebrochen wird.

Gemäß einem vorteilhaften und gegebenenfalls separat zu beanspruchenden Aspekt der Erfindung ist das Aufzeichnungsmittel in der Ventilbrechvorrichtung integriert und / oder fest verbaut. Das Aufzeichnungsmittel kann mit der Ventilbrechvorrichtung zudem in einem gemeinsamen Gehäuse untergebracht sein.

Gegenüber einem modular zubaubaren oder auch einem komplett separaten Aufzeichnungsmittel hat eine derartige Integration des Aufzeichnungsmittels in die Ventilbrechvorrichtung den Vorteil, dass die Ausrichtung des Aufzeichnungsmittels relativ zu der Ventilbrechvorrichtung und der Position des Brechventils in der Ventilbrechvorrichtung stets gleich bleibt und daher nicht nachjustiert werden muss. Gemäß einem vorteilhaften und gegebenenfalls separat zu beanspruchenden Aspekt der Erfindung ist die Ventilbrechvorrichtung zudem fest verbaut und / oder stationär und somit an einen festen Standort gebunden. In anderen Worten ist die erfindungsgemäße Ventilbrechvorrichtung also fest installiert, z.B. als Teil eines Infusionssystems neben einem Patientenbett oder als Teil einer Blutaufbereitungsanlage oder einer Blutbehandlungsanlage und nicht mobil oder als Teil einer Handheld-Vorrichtung.

Grundsätzlich kann die erfindungsgemäße Ventilbrechvorrichtung aber auch mobil / tragbar sein.

Ein weiterer vorteilhafter und gegebenenfalls separat zu beanspruchender Aspekt der Erfindung betrifft ein Verfahren zum Brechen eines Brechventils einer medizinischen Schlauchleitung mit den Schritten:
Einbringen des zu brechenden Brechventils in eine Ventilbrechvorrichtung, vorzugsweise eine vorstehend beschriebene Ventilbrechvorrichtung;
während eines gleichzeitigen vorzugsweise kontaktlosen Auslesens eines an der medizinischen Schlauchleitung und / oder einem der medizinischen Schlauchleitung zugehörigen Behältnis angeordneten Identifikationselements;
Brechen des Brechventils durch eine kontrollierte gegenläufige Bewegung zweier entlang einer Longitudinalachse des Brechventils voneinander beabstandeter Abschnitte des Brechventils;
während einer gleichzeitigen Aufzeichnung von Daten bezüglich des Brechens des Brechventils mittels eines Aufzeichnungsmittels;
Bestimmung auf der Grundlage der von dem Aufzeichnungsmittel aufgezeichneten Daten, ob das Brechventil vollständig gebrochen wurde; und
wenn die Bestimmung ergibt, dass das Brechventil vollständig gebrochen ist, kontrolliertes Abbrechen der Bewegung mindestens eines der zwei voneinander beabstandeten Abschnitte des Brechventils, oder
wenn die Bestimmung ergibt, dass das Brechventil unvollständig gebrochen ist, kontrolliertes Weiterführen der Bewegung mindestens eines der zwei voneinander beabstandeten Abschnitte des Brechventils, bis eine erneute Bestimmung ergibt, dass das Brechventil vollständig gebrochen ist.

Das Identifikationselement kann hierbei ein Barcode, Transponder oder RFID-Chip oder auch ein einfaches Etikett sein.

Die zwei entlang einer Longitudinalachse des Brechventils voneinander beabstandeten Abschnitte des Brechventils können vorzugsweise ein erster und ein zweiter Endabschnitt des Brechventils sein.

Gemäß einem weiteren vorteilhaften, gegebenenfalls separat zu beanspruchenden Aspekt der Erfindung erfolgt die Bestimmung, ob das Brechventil vollständig gebrochen wurde, durch einen Abgleich der von dem Aufzeichnungsmittel aufgezeichneten Daten mit im Vorhinein bestimmten Referenzdaten.

Die Aufzeichnung von Daten bezüglich des Brechens des Brechventils mittels des Aufzeichnungsmittels kann kontinuierlich oder intermittierend zu einstellbaren Zeitpunkten erfolgen.

### Figurenbeschreibung

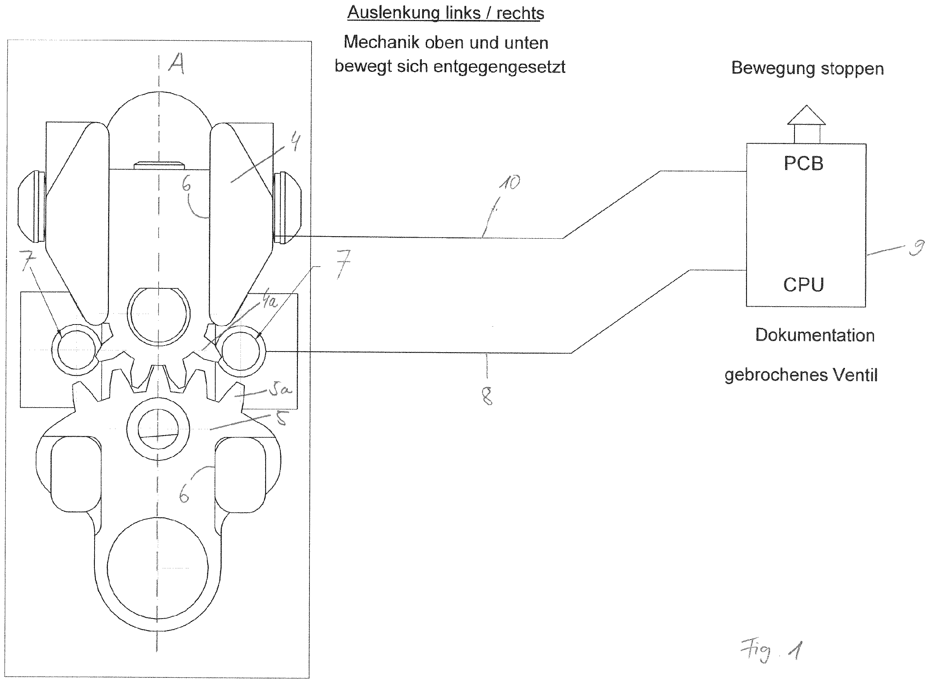

Im Folgenden werden einzelne Aspekte der vorliegenden Erfindung unter Bezugnahme auf die Figuren 1 bis 4 näher beschrieben.

In **Figur 1** ist der grundsätzliche Aufbau einer erfindungsgemäßen Ventilbrechvorrichtung 1 gezeigt. Die Ventilbrechvorrichtung 1 weist ein erstes Drehelement 4 und ein zweites Drehelement 5 auf. Das erste Drehelement 4 und das zweite Drehelement 5 weisen jeweils eine Aufnahme 6 auf, in welcher eine Schlauchleitung 2 reibschlüssig aufgenommen bzw. eingeführt werden kann.

Das erste Drehelement 4 ist mit einer Verzahnung / einem Zahnradteil 4a ausgestattet, welcher sich im Eingriff befindet mit einer korrespondierenden Verzahnung / einem korrespondierenden Zahnradteil 5a des zweiten Drehelements 5. In der in Fig. 1 gezeigten Ausführungsform ist die Wirkverbindung zwischen dem ersten Drehelement 4 und dem zweiten Drehelement 5 eine Verzahnung bzw. der Eingriff zweier Zahnradteile 4a und 5a.

Wird beispielsweise das erste Drehelement 4 im Uhrzeigersinn von der Longitudinalachse der Ventilbrechvorrichtung A ausgelenkt bzw. verschwenkt, so bewirkt der Zahnradeingriff zwischen der Verzahnung 4a des ersten Drehelements 4 und der Verzahnung 5a des zweiten Drehelements 5 eine gegenläufige Bewegung des zweiten Drehelements 5, d.h. das zweite Drehelement 5 wird im Gegenuhrzeigersinn von der Longitudinalachse A der Ventilbrechvorrichtung ausgelenkt bzw. verschwenkt.

Je nach Ausgestaltung der Verzahnungen 4a und 5a kann die Bewegung des zweiten Drehelements 5 entgegengesetzt und gleich, aber auch kleiner oder größer sein als die Bewegung des ersten Drehelements 4. Durch die Einstellung des Übersetzungsverhältnisses zwischen den Zahnradteilen 4a und 5a lässt sich das Verhältnis der Bewegung des ersten Drehelements 4 zu der Bewegung des zweiten Drehelements 5 einstellen.

Entlang der Longitudinalachse A der Ventilbrechvorrichtung 1 sind zwischen dem ersten Drehelement 4 und dem zweiten Drehelement 5 zwei Kameras 7 angeordnet. Die Kameras / sind im Wesentlichen auf Höhe des Wirkeingriffs zwischen dem ersten Drehelement 4 und dem zweiten Drehelement 5 angeordnet.

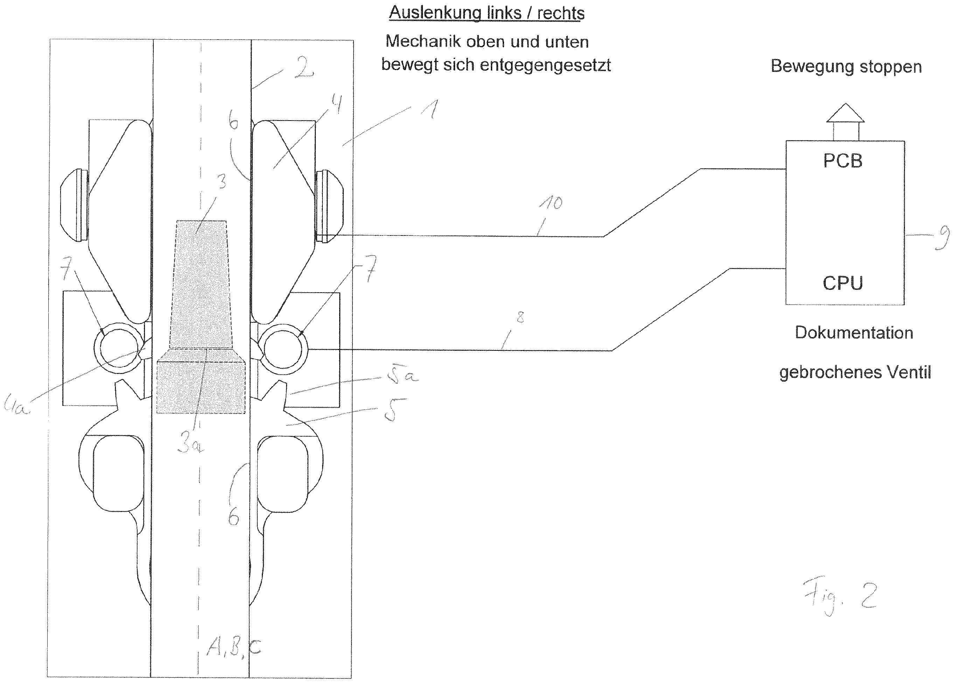

**Figur 2** zeigt die erfindungsgemäße Ventilbrechvorrichtung 1 aus Fig. 1 mit einer in die Ventilbrechvorrichtung 1 eingeführten Schlauchleitung 2 im Ruhezustand.

In der Schlauchleitung 2 ist ein zu brechendes Brechventil 3 mit einer Sollbruchstelle 3a. Die Ventilbrechvorrichtung 1 weist ein erstes Drehelement 4 und ein zweites Drehelement 5 auf. Das erste Drehelement 4 und das zweite Drehelement 5 weisen jeweils eine Aufnahme 6 auf, in welcher in der Darstellung nach Fig. 1 die Schlauchleitung 2 reibschlüssig aufgenommen ist.

Das erste und / oder das zweite Drehelement 4, 5 ist vorteilhafterweise aus einem Kunststoffmaterial gefertigt, sodass eine Beschädigung der aufgenommenen Schlauchleitung durch scharfe Kanten vermieden wird. Alternativ kann das erste und / oder das zweite Drehelement auch aus Metall gefertigt sein.

Das erste Drehelement 4 ist mit einer Verzahnung / einem Zahnradteil 4a ausgestattet, welcher sich im Eingriff mit einer korrespondierenden Verzahnung / einem korrespondierenden Zahnradteil 5a des zweiten Drehelements 5 befindet.

Durch den Eingriff der Verzahnungen 4a und 5a des ersten Drehelements 4 und des zweiten Drehelements 5 bewirkt eine Bewegung beispielsweise des ersten Drehelements 4 eine gegenläufige Bewegung des Drehelements 5.

Entlang der Longitudinalachse A der Ventilbrechvorrichtung 1 sind zwischen dem ersten Drehelement 4 und dem zweiten Drehelement 5 zwei Kameras 7 angeordnet. Die Kameras 7 sind jeweils entlang der Longitudinalachse A der Ventilbrechvorrichtung auf der Höhe der Sollbruchstelle 3a des zu brechenden Brechventils 3 angeordnet. Dies ermöglicht eine optimale Überwachung und Dokumentation des Zustands der Sollbruchstelle 3a des Brechventils 3 während des Vorgangs des Brechens des Brechventils 3 durch die Ventilbrechvorrichtung 1.

Die Kameras 7 sind beispielsweise CCD-Kameras, welche beidseitig zu der Sollbruchstelle 3a angeordnet sind. Es wäre aber auch denkbar, eine Mehrzahl von Kameras 7 als ein die Schlauchleitung 2 auf Höhe der Sollbruchstelle 3a umlaufender Ring anzuordnen. Bei einer derartigen Anordnung könnte der Bruch der Sollbruchstelle 3a sogar noch schneller optisch erfasst werden, da sich zumindest eine der Mehrzahl der Kameras 7 stets in der optimalen Aufnahmeposition befindet.

Die Kameras 7 sind mittels einer Datenleitung 8 mit einer CPU (Central Processing Unit) 9 verbunden. Die CPU 9 ist wiederum mittels einer Datenleitung mit einem in der Figur 1 nicht gezeigten Antrieb, beispielsweise in Form eines Elektromotors oder sonstigen Motors, des ersten Drehelements 4 verbunden.

Die von der Kamera 7 aufgezeichneten Bilddaten werden bei einem Betrieb der Ventilbrechvorrichtung 1 über eine Datenleitung 8 an die CPU 9 weitergeleitet. In der CPU 9 findet ein Abgleich der von der Kamera 7 aufgezeichneten Bilddaten mit bereits im Vorhinein gespeicherten Bilddaten (Referenzbilddaten) eines vollständig gebrochenen Brechventils 3 gleichen Typs wie das zu diesem Zeitpunkt in die Ventilbrechvorrichtung 1 eingeführte Brechventil 3 statt. Die CPU 9 ist daher dazu ausgelegt, einen derartigen Bildabgleich vorzunehmen. Die CPU 9 fungiert daher auch als Vergleichseinheit.

Zudem bietet die Aufzeichnung des Brechvorgangs durch die Kameras 7 den zusätzlichen Vorteil, dass der Vorgang des Brechens des Brechventils 3 in der Brechvorrichtung 1 lückenlos dokumentiert wird.

Die CPU 9 verfügt über einen Schaltkreis PCB (Printed circuit board) und bestimmt durch den vorstehend erläuterten Bildabgleich, ob das in der Ventilbrechvorrichtung 1 eingeführte Brechventil 3 vollständig gebrochen worden ist.

Ergibt der Bildabgleich, dass das Brechventil 3 vollständig gebrochen ist, so steuert die CPU 9 das erste Drehelement 4 über eine Datenleitung 10 dazu an, dass die Bewegung des Drehelements 4 gestoppt wird. Eine weitere Bewegung des ersten Drehelements 4 ist unnötig, da die Bewegung des ersten Drehelements 4 und somit die über den Zahnradeingriff bewirkte gegenläufige Bewegung des zweiten Drehelements 5 nur dem Zweck des Brechens des Brechventils 3 dient.

Die CPU 9 führt somit eine Rückkoppelungsregelung der Bewegung des ersten Drehelements 4 auf der Grundlage der von den Kameras 7 aufgezeichneten Bilder der Sollbruchstelle 3a des Brechventils 3 durch. Dadurch wird gewährleistet, dass das Drehelement 4 nur solange bewegt wird, bis das Brechventil 3 vollständig gebrochen ist; aber auch, dass das Drehelement ausreichend lange und weit bewegt wird, um das Brechventil 3 vollständig zu brechen.

Dies ermöglicht es, bei dem Vorgang des Öffnens des Fließwegs der Schlauchleitung 2 Zeit einzusparen, da das Brechventil 3 bereits beim ersten Versuch vollständig gebrochen und somit der Fließweg der Schlauchleitung 2 freigeschaltet wird.

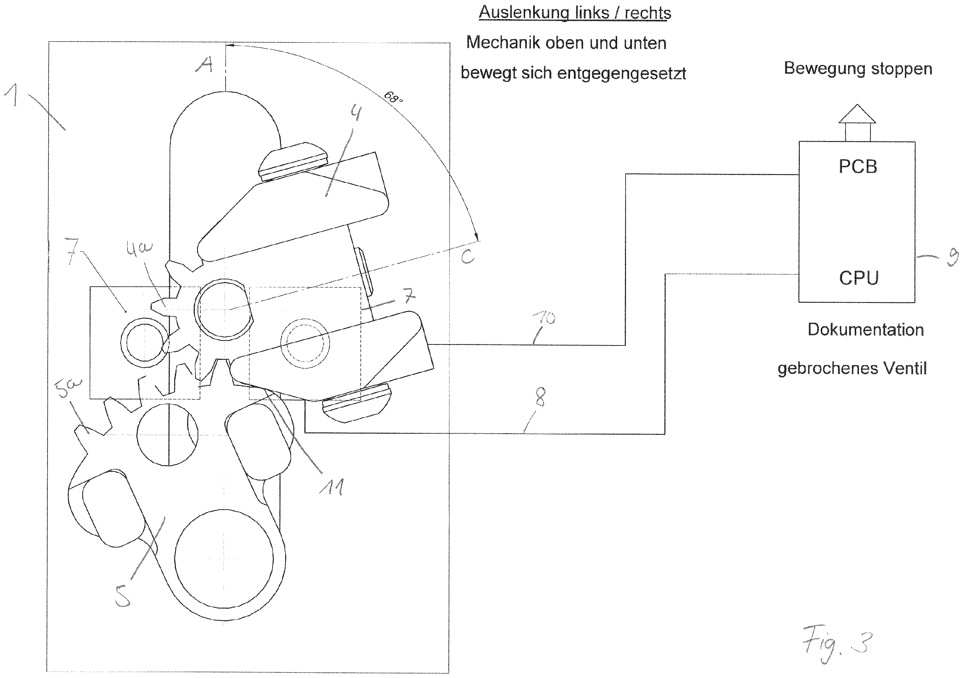

**Fig. 3** zeigt die erfindungsgemäße Ventilbrechvorrichtung 1 aus Fig. 1 im Aktivzustand. Durch die Auslenkung des ersten Drehelements 4 von der zentralen Longitudinalachse A der Ventilbrechvorrichtung 1 ist die zentrale Longitudinalachse C des ersten Drehelements 4 um 68° von der zentralen Longitudinalachse A der Ventilbrechvorrichtung 1 ausgelenkt.

Das erste Drehelement 4, d.h. auch die Longitudinalachse C des ersten Drehelements 4, ist von der zentralen Longitudinalachse A der Ventilbrechvorrichtung 1 um 68° im Uhrzeigersinn verschwenkt bzw. ausgelenkt. In der Figur 3 ist zur Verdeutlichung der 68° - Winkel zwischen den Achsen A und C eingezeichnet.

In der Figur 3 ist zudem der Zahnradeingriff zwischen der Verzahnung 4a des ersten Drehelements 4 und der Verzahnung 5a des zweiten Drehelements 5 deutlich zu sehen.

Durch einen Anschlag zwischen dem ersten Drehelement 4 und dem zweiten Drehelement 5 kann zudem die maximale Auslenkung des ersten Drehelements 4 von der zentralen Longitudinalachse A begrenzt werden. Bei der in der Fig. 3 gezeigten Ausführungsform beträgt die maximale Auslenkung 68°, bei diesem Winkel entsteht ein Kontakt in dem Anschlagspunkt bzw. Kontaktpunkt 11 zwischen dem ersten Drehelement 4 und dem zweiten Drehelement 5.

Das heißt, in dieser Position kommt das erste Drehelement 4 auf dem zweiten Drehelement 5 zu liegen, wodurch eine weitere Auslenkung des ersten Drehelements 4 im Uhrzeigersinn blockiert wird.

Je nach Anwendung kann der Kontaktpunkt 11 auch mit einem Vorsprung auf dem ersten Drehelement 4 und / oder dem zweiten Drehelement 5 ausgestattet werden, sodass sich der Maximalwert der Auslenkung einstellen lässt.

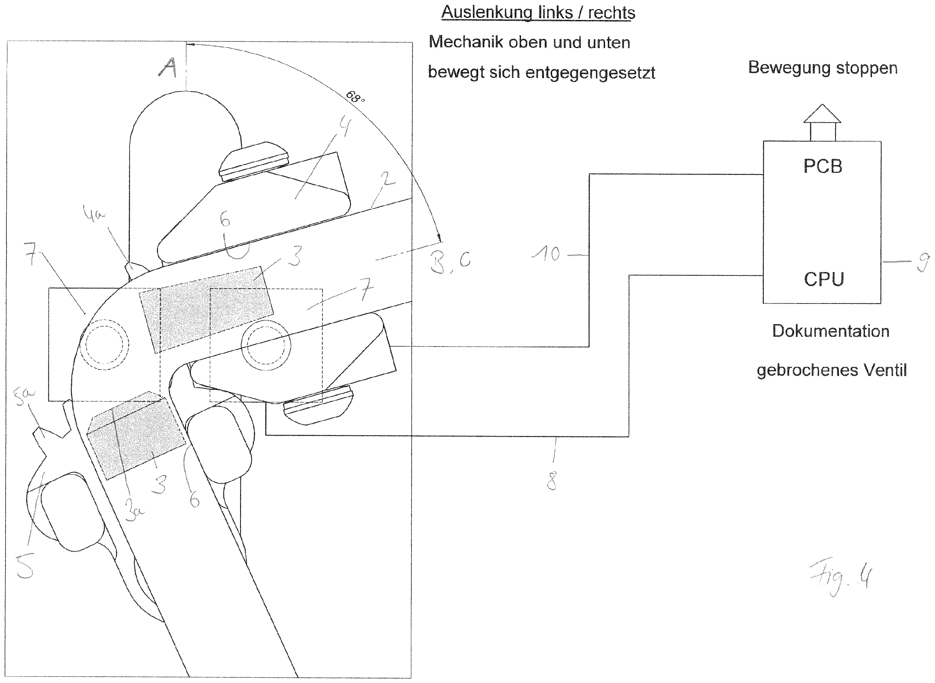

**Fig. 4** zeigt die erfindungsgemäße Ventilbrechvorrichtung 1 aus Fig. 2 mit einer in die Ventilbrechvorrichtung 1 eingeführten Schlauchleitung 2 im Aktivzustand. Durch die Auslenkung des ersten Drehelements 4 von der zentralen Longitudinalachse A der Ventilbrechvorrichtung 1 wurde die zentrale Longitudinalachse B der in der Ventilbrechvorrichtung 1 aufgenommenen Schlauchleitung 2 und auch die Longitudinalachse C des ersten Drehelements 4 um 68° von der zentralen Longitudinalachse A der Ventilbrechvorrichtung 1 ausgelenkt.

In dem in Fig. 2 gezeigten Ruhezustand der Ventilbrechvorrichtung 1 fallen die Achsen A und B und C zusammen.

In dem in Fig. 4 gezeigten Aktivzustand ist zudem deutlich erkennbar, dass auch das zweite Drehelement 5 von der zentralen Longitudinalachse A der Ventilbrechvorrichtung verschwenkt bzw. ausgelenkt ist. Konkret ist das erste Drehelement 4 relativ zu der Achse A im Uhrzeigersinn, d.h. nach rechts, ausgelenkt und das zweite Drehelement 5 ist relativ zu der Achse A im Gegenuhrzeigersinn, d.h. nach links ausgelenkt.

In der Darstellung in Fig. 4 ist das Brechventil 3 durch die Sollbruchstelle 3a in einen oberen Abschnitt und einen unteren Abschnitt geteilt.

Durch die Auslenkung des ersten Drehelements 4 und des zweiten Drehelements 5 sind auch der obere Abschnitt und der untere Abschnitt des Brechventils 3 bewegt bzw. verschoben worden. Der obere Abschnitt des Brechventils 3 ist hierbei im Uhrzeigersinn ausgelenkt worden und der untere Abschnitt des Brechventils ist im Gegenuhrzeigersinn ausgelenkt worden, bis das Brechventil 3 an seiner Sollbruchstelle 3a gebrochen ist.

In der in Fig. 4 gezeigten Position ist das zu brechende Brechventil 3 an seiner Sollbruchstelle 3a vollständig gebrochen. Zu diesem Zeitpunkt, d.h. sobald die Kameras 7 ein Bild des vollständig gebrochenen Brechventil 3 aufgezeichnet haben und dieses an die CPU 9 weitergeleitet haben, wird in der CPU durch einen Bildabgleich mit Referenzdaten bestimmt, dass das Brechventil 3 vollständig gebrochen ist.

Daraufhin sendet die CPU 9 über eine Datenleitung 10 ein Steuerungssignal an einen Antrieb, z.B. einen Motor, des ersten Drehelements 4, sodass die Auslenkbewegung des ersten Drehelements 4 gestoppt wird.

Solange nicht bestimmt worden ist, dass das Brechventil 3 vollständig gebrochen ist, wird das erste Drehelement 4 weiter ausgelenkt, bis die maximale Auslenkung durch eine Blockade einer weiteren Auslenkung durch den Kontaktpunkt 11 erreicht ist, d.h. bis der mögliche Maximalwert der Auslenkung erreicht ist.

## Patentansprüche

1. Ventilbrechvorrichtung (1) zum Brechen eines Brechventils (3) einer medizinischen Schlauchleitung (2), mit:
einem ersten Drehelement (4), welches relativ zu einer zentralen Longitudinalachse (A) der Ventilbrechvorrichtung (1) bewegbar ist, und
einem zweiten Drehelement (5), welches relativ zu der zentralen Longitudinalachse (A) der Ventilbrechvorrichtung (1) bewegbar ist, und
einem Aufzeichnungsmittel (7) zur Überwachung und Aufzeichnung des Vorgangs des Brechens des Brechventils (3),
**dadurch gekennzeichnet, dass**
das zweite Drehelement (5) mit dem ersten Drehelement (4) derart in Wirkverbindung steht, dass eine Bewegung des ersten Drehelements (4) in einer ersten Richtung eine Bewegung des zweiten Drehelements (5) in einer zweiten Richtung bewirkt, wobei die erste Richtung zu der zweiten Richtung gegenläufig ist.

2. Ventilbrechvorrichtung (1) nach Anspruch 1, **dadurch gekennzeichnet, dass** das erste Drehelement (4) aktiv bewegbar ist und das zweite Drehelement (5) ausschließlich durch die Bewegung des ersten Drehelements (4) passiv bewegt wird.

3. Ventilbrechvorrichtung (1) nach einem der vorhergehenden Ansprüche, **dadurch gekennzeichnet, dass** die Wirkverbindung zwischen dem ersten und dem zweiten Drehelement (4,5) ein Zahnradeingriff ist.

4. Ventilbrechvorrichtung (1) nach einem der vorhergehenden Ansprüche, **dadurch gekennzeichnet, dass** das Aufzeichnungsmittel (7) zwischen dem ersten und dem zweiten Drehelement (4,5) auf der Höhe des Wirkeingriffs zwischen dem ersten und dem zweiten Drehelement (4,5) angeordnet ist.

5. Ventilbrechvorrichtung (1) nach einem der vorhergehenden Ansprüche, **dadurch gekennzeichnet, dass** das Aufzeichnungsmittel (7) mindestens eine Kamera umfasst.

6. Ventilbrechvorrichtung (1) nach einem der vorhergehenden Ansprüche, **dadurch gekennzeichnet, dass** das Aufzeichnungsmittel (7) dazu ausgelegt ist, Daten bezüglich des Vorgangs des Brechens des Brechventils (3) aufzuzeichnen, und die Ventilbrechvorrichtung (1) weiterhin eine Steuerungseinheit aufweist, welche dazu ausgelegt ist, die von dem Aufzeichnungsmittel (7) aufgezeichneten Daten mit im Vorhinein bestimmten Referenzdaten abzugleichen, um dadurch zu bestimmen, ob das Brechventil (3) vollständig gebrochen ist.

7. Ventilbrechvorrichtung (1) nach Anspruch 6, **dadurch gekennzeichnet, dass** die Steuerungseinheit weiterhin dazu ausgelegt ist,
- eine Bewegung mindestens des ersten Drehelements (4) abzubrechen,
wenn durch den Abgleich der von dem Aufzeichnungsmittel (7) ermittelten Daten mit den Referenzdaten bestimmt worden ist, dass das Brechventil (3) vollständig gebrochen ist; oder
- eine Bewegung mindestens des ersten Drehelements (4) weiterzuführen,
wenn durch den Abgleich der von dem Aufzeichnungsmittel (7) ermittelten Daten mit den Referenzdaten bestimmt worden ist, dass das Brechventil (3) unvollständig gebrochen ist.

8. Ventilbrechvorrichtung nach einem der vorhergehenden Ansprüche, **dadurch gekennzeichnet, dass** das Aufzeichnungsmittel (7) in der Ventilbrechvorrichtung integriert und / oder fest verbaut ist.

9. Ventilbrechvorrichtung nach einem der vorhergehenden Ansprüche, **dadurch gekennzeichnet, dass** die Bewegung des ersten und / oder des zweiten Drehelements (4,5) eine Auslenkung relativ zu der zentralen Longitudinalachse (A) der Ventilbrechvorrichtung (1) ist.

10. Ventilbrechvorrichtung (1) nach einem der vorhergehenden Ansprüche, **weiterhin gekennzeichnet durch** ein Begrenzungsmittel (11), das dazu ausgelegt ist, einen Maximalwert eines Bewegungsbereichs mindestens des ersten Drehelements (4) festzusetzen.

11. Ventilbrechvorrichtung (1) nach einem der vorhergehenden Ansprüche, **dadurch gekennzeichnet, dass** die Ventilbrechvorrichtung (1) fest verbaut und / oder stationär ist und somit an einen festen Standort gebunden ist.

12. Verfahren zum Brechen eines Brechventils (3) einer medizinischen Schlauchleitung (2),
mit den Schritten:
- Einbringen des zu brechenden Brechventils (3) in eine Ventilbrechvorrichtung (1) nach einem der Ansprüche 1 bis 11; während eines
- gleichzeitigen Auslesens eines an der medizinischen Schlauchleitung (2) und / oder einem der medizinischen Schlauchleitung (2) zugehörigen Behältnis angeordneten Identifikationselements;
- Brechen des Brechventils (3) durch eine kontrollierte gegenläufige Bewegung zweier entlang einer Longitudinalachse des Brechventils (3) voneinander beabstandeter Abschnitte des Brechventils (3); während einer
- gleichzeitigen Aufzeichnung von Daten bezüglich des Vorgangs des Brechens des Brechventils (3) mittels eines Aufzeichnungsmittels (7);
- Bestimmung auf der Grundlage der von dem Aufzeichnungsmittel (7) aufgezeichneten Daten, ob das Brechventil (3) vollständig gebrochen wurde; und
wenn die Bestimmung ergibt, dass das Brechventil (3) vollständig gebrochen ist,
- kontrolliertes Abbrechen der Bewegung mindestens eines der zwei voneinander beabstandeten Abschnitte des Brechventils (3), oder
wenn die Bestimmung ergibt, dass das Brechventil (3) unvollständig gebrochen ist,
- kontrolliertes Weiterführen der Bewegung mindestens eines der zwei voneinander beabstandeten Abschnitte des Brechventils (3), bis eine erneute Bestimmung ergibt, dass das Brechventil (3) vollständig gebrochen ist.

13. Verfahren nach Anspruch 12, **dadurch gekennzeichnet, dass** die Bestimmung, ob das Brechventil (3) vollständig gebrochen wurde, durch einen Abgleich der von dem Aufzeichnungsmittel (7) aufgezeichneten Daten mit im Vorhinein bestimmten Referenzdaten erfolgt.

14. Verfahren nach einem der Ansprüche 12 oder 13, **dadurch gekennzeichnet, dass** die Aufzeichnung von Daten bezüglich des Vorgangs des Brechens des Brechventils (3) mittels eines Aufzeichnungsmittels (7) kontinuierlich oder intermittierend zu einstellbaren Zeitpunkten erfolgt.

## Claims

1. A valve breaking device (1) for breaking a breaking valve (3) of a medical hose line (2), comprising:
a first rotary member (4) movable relative to a central longitudinal axis (A) of the valve breaking device (1), and
a second rotary member (5) movable relative to the central longitudinal axis (A) of the valve breaking device (1); and
a recording means (7) for monitoring and recording the process of breaking the breaking valve (3),
**characterized in that**
the second rotary member (5) is engaged with the first rotary member (4) such that a movement of the first rotary member (4) in a first direction causes a movement of the second rotary member (5) in a second direction, the first direction being opposite to the second direction.

2. The valve breaking device (1) according to claim 1, **characterized in that** the first rotary member (4) is actively movable and the second rotary member (5) is passively moved exclusively by the movement of the first rotary member (4).

3. The valve breaking device (1) according to one of the preceding claims, **characterized in that** the engagement between the first and the second rotary element (4, 5) is a gear engagement.

4. The valve breaking device (1) according to one of the preceding claims, **characterized in that** the recording means (7) is disposed between the first and second rotary members (4,5) at the level of the engagement between the first and second rotary members (4,5).

5. The valve breaking device (1) according to one of the preceding claims, **characterized in that** the recording means (7) comprises at least one camera.

6. The valve breaking device (1) according to one of the preceding claims, **characterized in that** the recording means (7) is adapted to record data relating to the process of breaking the breaking valve (3), and the valve breaking device (1) further comprises a control unit adapted to compare the data recorded by the recording means (7) with reference data determined in advance to thereby determine whether the breaking valve (3) is completely broken.

7. The valve breaking device (1) according to claim 6, **characterized in that** the control unit is further adapted to,
- abort a movement of at least the first rotary element (4),
when it has been determined, by comparing the data determined by the recording means (7) with the reference data, that the breaking valve (3) is completely broken; or
- continue a movement of at least the first rotary element (4),
when it has been determined, by comparing the data determined by the recording means (7) with the reference data, that the breaking valve (3) is incompletely broken.

8. The valve breaking device according to one of the preceding claims, **characterized in that** the recording means (7) is integrated and/or fixedly installed in the valve breaking device.

9. The valve breaking device according to one of the preceding claims, **characterized in that** the movement of the first and/or the second rotary element (4, 5) is a deflection relative to the central longitudinal axis (A) of the valve breaking device (1).

10. The valve breaking device (1) according to one of the preceding claims, **further characterized by** a limiting means (11) adapted to set a maximum value of a range of movement of at least the first rotary member (4).

11. The valve breaking device (1) according to one of the preceding claims, **characterized in that** the valve breaking device (1) is fixedly installed and/or stationary and is thus bound to a fixed location.

12. A method for breaking a breaking valve (3) of a medical hose line (2),
comprising the steps:
- inserting the breaking valve (3) to be broken into a valve breaking device (1) according to any one of claims 1 to 11; while simultaneously the step of
- reading an identification element disposed on the medical hose line (2) and/or a container associated with the medical hose line (2);
- breaking the breaking valve (3) by a controlled counter-rotating movement of two portions of the breaking valve (3) spaced apart from each another along a longitudinal axis of the breaking valve (3); while simultaneously performing the step of
- recording data relating to the operation of breaking the breaking valve (3) by means of a recording means (7);
- determining, on the basis of the data recorded by the recording means (7), whether the breaking valve (3) has been completely broken; and
when the determination indicates that the breaking valve (3) is completely broken,
- controlled aborting of the movement of at least one of the two spaced portions of the break valve (3), or
if the determination indicates that the breaking valve (3) is incompletely broken,
- controlled continuing of the movement of at least one of the two spaced apart portions of the breaking valve (3) until a renewed determination indicates that the breaking valve (3) is completely broken.

13. The method according to claim 12, **characterized in that** the determination whether the breaking valve (3) has been completely broken is made by comparing the data recorded by the recording means (7) with reference data determined in advance.

14. The method according to one of the claims 12 or 13, **characterized in that** the recording of data related to the process of breaking the breaking valve (3) is performed continuously or intermittently at adjustable times by means of a recording means (7).

## Revendications

1. Dispositif de rupture de valve (1) destiné à rompre une valve pouvant être rompue (3) d'une conduite flexible médicale (2), avec :
un premier élément rotatif (4), lequel est mobile par rapport à un axe longitudinal central (A) du dispositif de rupture de valve (1), et
un deuxième élément rotatif (5), lequel est mobile par rapport à l'axe longitudinal central (A) du dispositif de rupture de valve (1), et
un moyen d'enregistrement (7) destiné à surveiller et à enregistrer le processus de rupture de la valve pouvant être rompue (3),
**caractérisé en ce que**
le deuxième élément rotatif (5) est en liaison fonctionnelle avec le premier élément rotatif (4) de telle sorte qu'un mouvement du premier élément rotatif (4) dans une première direction provoque un mouvement du deuxième élément rotatif (5) dans une deuxième direction, dans lequel la première direction est contraire à la deuxième direction.

2. Dispositif de rupture de valve (1) selon la revendication 1, **caractérisé en ce que** le premier élément rotatif (4) est mobile de manière active et le deuxième élément rotatif (5) est déplacé de manière passive exclusivement au moyen du mouvement du premier élément rotatif (4).

3. Dispositif de rupture de valve (1) selon l'une quelconque des revendications précédentes, **caractérisé en ce que** la liaison fonctionnelle entre le premier et le deuxième élément rotatif (4, 5) est un engrènement de roues dentées.

4. Dispositif de rupture de valve (1) selon l'une quelconque des revendications précédentes, **caractérisé en ce que** le moyen d'enregistrement (7) est disposé entre le premier et le deuxième élément rotatif (4, 5) au niveau de la prise fonctionnelle entre le premier et le deuxième élément rotatif (4, 5).

5. Dispositif de rupture de valve (1) selon l'une quelconque des revendications précédentes, **caractérisé en ce que** le moyen d'enregistrement (7) comporte au moins une caméra.

6. Dispositif de rupture de valve (1) selon l'une quelconque des revendications précédentes, **caractérisé en ce que** le moyen d'enregistrement (7) est conçu pour enregistrer des données concernant le processus de rupture de la valve pouvant être rompue (3), et le dispositif de rupture de valve (1) présente en outre une unité de commande, laquelle est conçue pour comparer les données enregistrées par le moyen d'enregistrement (7) à des données de référence préalablement déterminées, afin de déterminer de cette façon si la valve pouvant être rompue (3) est entièrement rompue.

7. Dispositif de rupture de valve (1) selon la revendication 6, **caractérisé en ce que** l'unité de commande est conçue en outre
- pour interrompre un mouvement au moins du premier élément rotatif (4), lorsqu'il a été déterminé au moyen de la comparaison des données déterminées par le moyen d'enregistrement (7) aux données de référence que la valve pouvant être rompue (3) est entièrement rompue ; ou
- pour poursuivre un mouvement d'au moins le premier élément rotatif (4), lorsqu'il a été déterminé au moyen de la comparaison des données déterminées par le moyen d'enregistrement (7) aux données de référence que la valve pouvant être rompue (3) est entièrement rompue.

8. Dispositif de rupture de valve selon l'une quelconque des revendications précédentes, **caractérisé en ce que** le moyen d'enregistrement (7) est intégré et/ou monté solidement dans le dispositif de rupture de valve.

9. Dispositif de rupture de valve selon l'une quelconque des revendications précédentes, **caractérisé en ce que** le mouvement du premier et/ou du deuxième élément rotatif (4, 5) est une déviation par rapport à l'axe longitudinal central (A) du dispositif de rupture de valve (1).

10. Dispositif de rupture de valve (1) selon l'une quelconque des revendications précédentes, **caractérisé en outre par** un moyen de limitation (11), qui est conçu pour fixer une valeur maximale d'une plage de mouvement au moins du premier élément rotatif (4).

11. Dispositif de rupture de valve (1) selon l'une quelconque des revendications précédentes, **caractérisé en ce que** le dispositif de rupture de valve (1) est monté solidement et/ou fixe et est donc attaché à un emplacement permanent.

12. Procédé de rupture d'une valve pouvant être rompue (3) d'une conduite flexible médicale (2),
comprenant les étapes :
- d'introduction de la valve pouvant être rompue (3) à rompre dans un dispositif de rupture de valve (1) selon l'une quelconque des revendications 1 à 11 ; pendant une
- lecture simultanée d'un élément d'identification disposé sur la conduite flexible médicale (2) et/ou un contenant associé à la conduite médicale flexible (2) ;
- de rupture de la valve pouvant être rompue (3) au moyen d'un mouvement contraire contrôlé de deux parties de la valve pouvant être rompue (3) espacées l'une de l'autre le long d'un axe longitudinal de la valve pouvant être rompue (3) ; pendant un
- enregistrement simultané de données concernant le processus de rupture de la valve pouvant être rompue (3) à l'aide d'un moyen d'enregistrement (7) ;
- du fait de déterminer sur la base des données enregistrées par le moyen d'enregistrement (7) si la valve pouvant être rompue (3) a été entièrement rompue ; et
s'il résulte de la détermination que la valve pouvant être rompue (3) est entièrement rompue,
- d'interruption contrôlée du mouvement d'au moins une des deux parties séparées l'une de l'autre de la valve pouvant être rompue (3), ou
s'il résulte de la détermination que la valve pouvant être rompue (3) est entièrement rompue,
- de poursuite contrôlée du mouvement au moins d'une des deux parties espacées l'une de l'autre de la valve pouvant être rompue (3), jusqu'à ce qu'il résulte d'une nouvelle détermination que la valve pouvant être rompue (3) est entièrement rompue.

13. Procédé selon la revendication 12, **caractérisé en ce que** le fait de déterminer si la valve pouvant être rompue (3) a été entièrement rompue s'effectue au moyen d'une comparaison des données enregistrées par le moyen d'enregistrement (7) à des données de référence déterminées préalablement.

14. Procédé selon l'une quelconque des revendications 12 ou 13, **caractérisé en ce que** l'enregistrement de données concernant le processus de rupture de la valve pouvant être rompue (3) s'effectue en continu ou par intermittence à instants réglables à l'aide d'un moyen d'enregistrement (7).
